(19) Europäisches Patentamt / European Patent Office / Office européen des brevets

(11) **EP 4 632 754 A1**

(12) **EUROPEAN PATENT APPLICATION**

(43) Date of publication:
**15.10.2025 Bulletin 2025/42**

(21) Application number: **24169585.7**

(22) Date of filing: **11.04.2024**

(51) International Patent Classification (IPC):
**G16H 20/30** (2018.01)   **A61B 5/00** (2006.01)
**G16H 50/20** (2018.01)

(52) Cooperative Patent Classification (CPC):
**G16H 20/30; A61B 5/4806; A61B 5/4812; G16H 50/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **KUHN, Jasmin**
  **Eindhoven (NL)**

• **SCHIPHORST, Laura Reina Bernarda**
  **Eindhoven (NL)**
• **DOS SANTOS DA FONSECA, Pedro Miguel Ferreira**
  **5656AG Eindhoven (NL)**
• **OVEREEM, Sebastiaan**
  **Eindhoven (NL)**
• **VAN GILST, Merel Marietje**
  **Eindhoven (NL)**

(74) Representative: **Philips Intellectual Property & Standards**
**High Tech Campus 52**
**5656 AG Eindhoven (NL)**

(54) **COMPUTER-IMPLEMENTED METHOD, COMPUTER PROGRAM PRODUCT, AND RESPIRATORY SUPPORT SYSTEM**

(57)    There is provided a computer-implemented method for determining sleep misperception of a subject during a sleep session. The method comprises receiving sleep condition information. The sleep condition information comprises at least one of SDB information and insomnia information. The SDB information is representative of a sleep disordered breathing (SDB) condition of the subject based on a number of SDB events of the subject per unit of time. The computer-implemented method comprises receiving sleep quality information representative of a quality of sleep of the sleep session experienced by the subject. The computer-implemented method comprises determining a degree of sleep misperception based on the sleep condition information and the sleep quality information. The sleep misperception is representative of a difference between a subjective total sleep time of the sleep session experienced by the subject and an objective total sleep time of the subject in the sleep session.

FIG. 1

EP 4 632 754 A1

**Description**

FIELD OF THE INVENTION

The invention relates to a computer-implemented method for determining sleep misperception of a subject during a sleep session. Further, the invention relates to a computer-implemented method for determining sleep quality of a sleep session. Further, the invention relates to a computer program product, comprising instructions which, when executed by a processing system, cause the processing system to carry out the computer-implemented methods. Further, the invention relates to a respiratory support system for providing pressurized air to a subject.

BACKGROUND OF THE INVENTION

Sleep-disordered breathing (SDB) conditions, such as obstructive sleep apnea (OSA) and central sleep apnea (CSA), are becoming increasingly common, and are particularly prevalent in older people, people with a high body mass index, smokers, heavy drinkers, and people with conditions such as coronary artery disease, hypertension and diabetes mellitus.

SDB conditions are commonly treated using positive airway pressure (PAP) therapy, in which pressurized air is provided to a subject to keep the subject's airways open. When first prescribing PAP therapy, a PAP titration study is carried out for the subject in order to determine a level of airway pressure to be provided to the subject during PAP therapy, as well as a suitable PAP therapy modality (e.g. continuous positive airway pressure, CPAP, bilevel positive airway pressure, BiPAP, or automatic positive airway pressure, APAP) and a suitable subject interface (e.g. a nasal pillow, an oronasal/full-face mask).

Although PAP therapy provides an effective way to prevent the airway from collapsing during sleep, the PAP therapy causes discomfort that causes difficulties for many subjects to fall asleep or to remain asleep. More than 50% of the subjects stop with the PAP therapy within one year, while they still suffer from the SDB condition. Many subjects stop with the PAP therapy in an attempt to improve their amount of sleep time.

SUMMARY OF THE INVENTION

It is an objective of the invention to improve PAP therapy.

According to a first aspect, there is provided a computer-implemented method for determining sleep misperception of a subject during a sleep session. The computer-implemented method comprises receiving sleep condition information. The sleep condition information comprises at least one of SDB information and insomnia information. The SDB information is representative of a sleep disordered breathing (SDB) condition of the subject based on a number of SDB events of the subject per unit of time. The insomnia information is representative of insomnia of the subject. The computer-implemented method comprises receiving sleep quality information representative of a quality of sleep of the sleep session experienced by the subject. The computer-implemented method comprises determining a degree of sleep misperception based on the sleep condition information and the sleep quality information. The sleep misperception is representative of a difference between a subjective total sleep time of the sleep session experienced by the subject and an objective total sleep time of the subject in the sleep session.

The inventors have discovered that a degree of sleep misperception can be determined based on the sleep condition information and the sleep quality information. The sleep condition information is information about the sleep condition of the subject. In many cases, the sleep condition of the subject remains the same over a longer period, such as several months or several years. As a result, the sleep condition information may be obtained during a diagnostic sleep session at a sleep center. Such a diagnostic sleep session may occur, for example, every 6 months or every year. The sleep quality information is obtained in an easy way by having the subject answer a questionnaire after a sleep session at home. By combining the information from the diagnostic sleep session and the sleep quality information, the degree of sleep misperception is determined. The degree of sleep misperception is determined in an accurate way with a minimum of discomfort to the subject. The degree of sleep misperception is an indicator of the difficulty a subject has or is going to have undergoing PAP therapy. So by determining the degree of sleep misperception according to the invention, the subject and the professional caretaker can be timely made aware about any difficulties with the PAP therapy, and actions can be taken accordingly. As a result, PAP therapy is improved.

Sleep misperception is a discrepancy between a subjective total sleep time and an objective total sleep time. The subjective total sleep time is the subject thinks he/she is asleep. The objective total sleep time is the subject is really asleep. Sleep misperception is highly clinically relevant since subjects who believe that they are getting less sleep than they actually do, may become anxious about their perceived lack of sleep. Such anxiety may lead to a reduced quality of life and to the subject becoming averse to the PAP therapy.

Objective total sleep time can be accurately determined using Electroencephalography (EEG). However, EEG measurements are available via sleep centers and hospitals, but in most cases too costly and labor intensive to have available at home. Also, EEG measurements are cumbersome to the subject because of the many electrodes and wires attached to the subject. As a result, EEG measurements are not representative of a typical sleep session of the subject at home. Algorithms are commercially available that estimate the objective total sleep time based on surrogate measures, such as photoplethysmography (PPG) or body movements. Such surrogate measures may be used at home. However, most of these surrogate measures have only limited accuracy to determine the objective total sleep time, because the surrogate measures are validated on a healthy population, but not people with a SDB condition and/or insomnia. Also, the surrogate measures require some kind of wearable device to be worn by the subject during multiple nights to obtain the measurements. Wearing such a wearable device makes it more difficult for the subject to properly fall asleep and stay asleep. By determining the sleep misperception according to the invention, the sleep misperception can be determined at home in an easy way, without reducing the comfort for the subject.

The sleep condition information comprises SDB information, or insomnia information, or both SDB information and insomnia information. For example, the sleep condition information consists of SDB information, or consists of insomnia information, or consists of both SDB information and insomnia information.

The SDB information is representative of a sleep disordered breathing (SDB) condition of the subject based on a number of SDB events of the subject per unit of time. For example, the SDB information is representative of the subject having obstructive sleep apnea (OSA) or central sleep apnea (CSA). For example, the SDB events comprises events during which the airflow of the subject stops for longer than a certain period, such as longer than 10 seconds or 15 seconds or 20 seconds. For example, the SDB events comprises events during which the subject has a reduced airflow for longer than a certain period, such as longer than 10 seconds or 15 seconds or 20 seconds. For example, the number of SDB events of the subject per unit of time is based on an apnea-hypopnea index (AHI). AHI is an index to indicate the severity of sleep apnea. AHI is calculated by dividing the number of apnea events and hypopnea events by the number of hours of sleep. For example, the AHI represents whether the subject has mild OSA, moderate OSA or severe OSA.

The insomnia information is representative of insomnia of the subject and/or insomnia symptoms experienced by the subject. Insomnia is a sleep condition in which a subject has insomnia symptoms for an extended period of time, such as difficulty falling asleep and/or difficulty staying asleep and/or waking up early. These difficulties last for at least several weeks, for example, several months or more. For example, the subject has these difficulties for reoccurring periods of weeks during several years. For example, according to the International Classification of Sleep Disorder, ICSD, insomnia is classified as a sleep disturbance that is present for a period of 3 months. A subject suffering from insomnia sleeps less time than required to be fully rested after a sleep session. The presence of insomnia is, for example, determined by having the subject answer a questionnaire. Validated insomnia questionnaires are available, such as the Insomnia Severity Index. The Insomnia Severity Index has seven questions. Based on the answer to these questions, the Insomnia Severity Index outputs a severity of the insomnia as either no insomnia, subthreshold insomnia, moderate sever insomnia, and sever insomnia. The insomnia information comprises, for example, information about whether the subject has insomnia or does not have insomnia. The insomnia information comprises, for example, information about a severity of the insomnia, such as mild insomnia, moderate insomnia, and sever insomnia. For example, a subset of the questions of the Insomnia Severity Index is used, such as only the first three items. The first three items relate to a subject's difficulty falling asleep, difficulty staying asleep, and problems of waking up too early. For example, the insomnia information comprises information about insomnia symptoms experienced by the subject, such as difficulty falling asleep, difficulty staying asleep, or problems of waking up too early.

The SDB condition and the insomnia have an influence on the sleep misperception. Firstly, each of the SDB condition and the insomnia affect the objective total sleep time and the subjective total sleep time. SDB events or the SDB therapy that attempts to reduce the number of SDB events cause arousals that awaken the subject. However, the wake periods are typically short, such as only a few seconds, after which the subject falls back to sleep. As a result, the subject is not aware of being awake. So the arousals shorten the objective total sleep time. The arousals do not shorten the subjective total sleep time, because the subject is unaware of being awake. This may result in a sleep misperception of the subject believing the subjective total sleep time is more than the objective total sleep time. For insomnia, the subject remains awake for longer periods of time, such as several hours. The subject is aware of being awake. Because of the desire of the subject to fall asleep, the subject may have anxiety. The anxiety causes the awake periods to be experienced as being longer than they are in reality. This may result in a sleep misperception of the subject believing the subjective total sleep time is less than the objective total sleep time.

Both the SDB condition and the insomnia are sleep conditions that remain substantially the same over several weeks or several months or more. For example, SDB condition is caused by lifestyle choices, such as nutrition and exercising. Even if a lifestyle is changed abruptly, the effects of the change take several weeks to take place. For example, a lifestyle change may reduce the weight of the subject from being overweight towards a healthy weight. Losing the weight takes several weeks or months. For example, SDB condition is caused by a physical trait of the subject, such as a narrow airway, or fat distribution around the throat, or excessive muscle relaxation during sleep. Such physical traits may not change at all, or only slowly with age. For example, a mental disorder, such as depression or an anxiety disorder, causes or worsens insomnia. Such disorders require a long time to recover from. For example, insomnia is caused by stress or trauma, which may take a long time to recover from.

The sleep quality is a subjective metric that represents how the subject experienced the quality of the sleep in the sleep session. The sleep quality is, for example, determined by answering a questionnaire, such as the Pittsburgh Sleep Quality Index (PSQI) questionnaire, or the Consensus Sleep Diary (Carney et.al. 2012). For example, the questionnaire asks the subject to rank the sleep quality of the sleep session on a scale of 1-5 or 1-10, wherein 1 represents poor quality, and the highest number represents an excellent quality. For example, the scale has relative expressions such as very poor, poor, neutral, good, very good. Even though the sleep quality is a subject metric provided by the subject, the sleep quality information provides an accurate metric based on which the sleep misperception can be determined.

The degree of sleep misperception is, for example, expressed as the sleep misperception index. The sleep misperception index MI is calculated as follows:

$$MI = (objective\ TST - subjective\ TST)/objective\ TST \qquad (1)$$

wherein objective TST is the objective total sleep time, and wherein the subjective TST is the subjective total sleep time.

For example, the degree of sleep misperception is expressed as a ratio between the subjective total sleep time and the objective total sleep time. For example, the degree of misperception is expressed as difference between the subjective total sleep time and the objective total sleep time. For example, the degree of sleep misperception is expressed as a scale with qualitive values, such as too low - normal - too high.

By determining the degree of sleep misperception, the subject and the professional caretake are assisted in providing the PAP therapy to the subject. In case the degree of sleep misperception represents that the subject perceives to have enough sleep, the PAP therapy may continue without any adjustments. In another example, the PAP therapy is intensified to further reduce the SDB events of the subject per unit of time, causing an improvement of the health of the subject. Because the subject perceives to have enough sleep, the subject is able to withstand such intensified PAP therapy. On the other hand, in case the degree of sleep misperception represents that the subject perceives not having enough sleep, the PAP therapy may need to be adjusted to ensure the subject is able to continue with the PAP therapy. For example, the PAP therapy is adjusted to be less intense. It may be more beneficial for the subject to continue with less intense PAP therapy which lasts entire sleep sessions, compared to more intense PAP therapy which the subject only sustains part of the sleep sessions. It is noted that sleep misperception is not a mere preference of the subject on whether to continue with PAP therapy. Instead, sleep misperception is measurable metric providing information about how well a subject is able to cope with PAP therapy. The sleep misperception represents a status of the subject. This status enables to properly apply or adjust the PAP therapy.

In an embodiment, the insomnia information comprises information about the insomnia based on a subjective perception of the subject.

According to this embodiment, the insomnia information comprises information based on the subjective perception of the subject. The most common way to diagnose insomnia is to ask a subject about difficulties with sleeping, and about how they feel during the day because of those difficulties. The subject responses in a subjective way to these questions, for example, by answering that falling asleep is difficult or very difficult. For example, the subject responses that there are feelings of fatigue or malaise during the day. Based on subjective perception, sleep doctors diagnose and treat insomnia. The same type of information about the subjective perception of the subject is used, in this embodiment, to determine the sleep misperception. This allows for an improved accuracy of determining the sleep misperception.

In an embodiment, the computer-implemented method comprises receiving sleep stage information representative of sleep stages of the subject during the sleep session. The sleep stages comprise a wake sleep stage and at least one non-wake sleep stage. The method comprises determining the insomnia information based on the sleep stage information.

According to this embodiment, the method makes use of sleep stage information to determine the insomnia information. The sleep stage information determines all insomnia information or only part of the insomnia information. For example, the method combines the sleep stage information with the information based on the subjective perception of the subject to determine the insomnia information. The sleep stages represent different stages of the subject during the sleep session. At least one sleep stage represents that the subject is awake, i.e., the wake sleep stage. One or more sleep stages represent that the subject is asleep. For example, there is only a single sleep stage representing that the subject is asleep. In another example, there are two sleep stages representing that the subject is asleep. One sleep stage is a Rapid Eye Movement (REM) sleep stage, whereas the other sleep stage is a non-REM sleep stage. In another example, there are three sleep stages representing that the subject is asleep. One sleep stage is a REM sleep stage, one sleep stage is a light sleep stage, and one sleep stage is a deep sleep stage. For example, the light sleep stage comprises two different sleep stages, such as N1 sleep stage and N2 sleep stage. For example, the deep sleep stage is N3 sleep stage. For example, the sleep stage information is based on an automatic sleep stage classifier or based on manual sleep scoring.

Based on the sleep stage information, the insomnia information is determined. For example, based on the sleep stage information, the method determines how long it takes for the subject to fall asleep, how often and how long the subject is awake after sleep onset. For example, the method determines how many times the subject is awake after sleep onset for a certain period of time. The certain period of time is, for example, more than 1 minute or more than 5 minutes or more than 10 minutes. This way, the method determines the insomnia information. For example, the method determines the insomnia information by using the sleep stage information from one or multiple sleep sessions of the subject. For example, the multiple sleep sessions expand over a period of more than one week or more than one month.

In an embodiment, the computer-implemented method comprises receiving at least one physiological signal representative of a physiological parameter of the subject, and determining sleep stage information based on the at least one physiological signal.

According to this embodiment, a physiological signal is received. Based on the physiological signal, the sleep stage information is based. By using the physiological signal of the subject to determine the sleep stage information, it can be detected if the insomnia of the subject changes. For example, the insomnia becomes better or worse over time. The change of the insomnia affects the physiological parameter of the subject. The change in the physiological parameter is detected via the physiological signal.

The physiological signal is, for example, based on neurological signals of the subject. For example, the neurological signals are obtained via polysomnography (PSG), via electroencephalography (EEG), via electrooculography (EOG), via electromyography (EMG) or any combination of these. A sensor adapted to generate a sensor signal based on the neurological signals is, for example, mounted on a wearable device for the head or face, such as a headband. The sleep stage information is based on the neurological signals via use of an automated sleep stage classifier or via manual annotation.

In addition or alternatively to using neurological signals, the physiological signal is, for example, based on a surrogate measure of sleep. For example, the physiological signal comprises a cardiac signal. The cardiac signal is obtained with an appropriate sensor such as a reflective photoplethysmography (PPG) sensor, a transmissive PPG sensor or a remote PPG sensor, a ballistocardiographic sensor, or a seismocardiographic sensor. The reflective PPG sensor is, for example, arranged on the wrist or the face of the subject. The transmissive PPG sensor is, for example, arranged on the finger of the subject. The remote PPG sensor comprises, for example, an infrared camera. The ballistocardiographic sensor comprises, for example, an accelerometer or gyroscope attached to the body of the subject, or for example a pressure sensor mounted in the mattress or bed of the subject. The seismocardiographic sensor comprises, for example, an accelerometer mounted on the chest of the subject. The signals obtained by one or more of these sensors are, for example, used as input to a machine learning model trained to infer sleep stages. The input is, for example, based on manually crafted features correlating with sleep stages, such as a feature describing heart rate variability, or a feature of a time series describing heart rate progression during sleep (e.g. instantaneous heart rate). The input is, for example, input as raw data to the machine learning model.

In addition or alternatively to determining the sleep stage information as mentioned above, the sleep stage information is, for example, based on respiratory activity. Respiratory activity of the subject is indicative of changes in autonomic nervous system activity associated with different sleep stages. Respiratory activity is, for example, measured with a sensor adapted to measure airflow or adapted to measure chest movements. For example, a sensor adapted to measure airflow comprises an oral cannula, a nasal cannula and/or a thermistor. For example, a sensor adapted to measure chest movements comprises a respiratory inductance plethysmography belt to be worn around the thorax of the abdomen. For example, the respiratory activity is measured with a sensor adapted to measure a pressure or to measure an airflow in a respiratory support device.

For example, the sensor adapted to measure respiratory activity comprises a pressure sensor mounted on the bed or mattress. For example, the sensor adapted to measure respiratory activity comprises a Doppler radar positioned near the subject. For example, the sensor for measuring respiratory activity comprises an accelerometer or a gyroscope mounted on the thorax, the abdomen and/or sternum of the subject.

In an embodiment, determining the degree of sleep misperception comprises using a model. The model has an input and an output. The input comprises the sleep condition information and the sleep quality information. The output comprises the degree of sleep misperception. The model is based on a correlation between a reference input and a reference output. The reference input comprises sleep condition information about multiple subjects. The reference input comprises sleep quality information about multiple sleep sessions of the multiple subjects. The reference output comprises a degree of sleep misperception for each of the multiple sleep sessions of the multiple subjects.

According to this embodiment, the model is based on the correlation between a reference input and a reference output. As a result, when providing the sleep condition information and the sleep quality information to the model, an accurate estimation of the degree of sleep misperception is created by the model.

In an embodiment, the model comprises a univariate linear mixed model.

According to this embodiment, the model comprises the univariate linear mixed model to estimate the degree of sleep misperception with improved accuracy. The univariate linear mixed model is univariate, because the model provides a single outcome variable, i.e., the sleep misperception. The sleep condition information has a constant effect in predicting the sleep misperception. The inventors have discovered that the relation between the sleep quality information and the sleep misperception is properly represented by a linear relationship. The univariate linear mixed model is a mixed model, because the model uses as a reference input information of multiple sleep sessions of a subject. For example, as reference input, information about 5 or 10 or 12 or 15 sleep sessions of a subject is used. For example, as reference input, information about multiple subjects is used. For example, the information about the multiple subjects relates to multiple sleep sessions of each of the multiple subjects. Each of the multiple subjects has the same number of sleep sessions, or some of the multiple subjects have more or less sleep sessions than other ones of the multiple subjects.

In an embodiment, the model comprises a machine learning model, wherein the machine learning model is at least one of a multi-level regression model, a Bayesian classifier, a logistic regression model, and a neural network.

According to this embodiment, the model comprises a machine learning model. The machine learning model is trained based on the reference input and the reference output.

In an embodiment, the computer -implemented method comprises receiving sleep time information representative of the objective total sleep time of the subject in the sleep session, and determining the sleep quality information based on the sleep time information and the insomnia information.

According to this embodiment, the inventors have discovered that the sleep quality can be determined based on the sleep time information and the insomnia information. As insomnia is a sleep condition that lasts for several weeks or longer, the insomnia severity information needs to be determined only occasionally, such as every month or every 6 months or every year. The sleep time information is obtained by obtaining the objective total sleep time of the subject during the sleep session. The objective total sleep time is the time the subject is asleep. The objective total sleep time can be obtained via unobtrusive measurements, such as PPG or ballistocardiography. So by using the insomnia information and the objective total sleep time, the sleep quality is determined without the need for the subject to use a sleep diary. This way, an improved way of determining sleep quality is provided.

In an embodiment, determining the sleep quality information comprises determining the sleep quality information based on the SDB information.

According to this embodiment, the SDB information is taken into account when determining the sleep quality information. The inventors have discovered that the SDB condition affects the sleep quality of the subject. The SDB condition is based on the number of SDB events of the subject per unit of time. In case the subject has a severe form of the SDB condition, the number of SDB events are high per unit of time. In case the subject has a mild form of the SDB condition, the number of SDB events are less high per unit of time. For example, in case the SDB condition is sleep apnea, the number of SDB events per unit of time may be represented by the Apnea-Hypopnea Index (AHI). AHI expresses the number of SDB events per hour. In case the subject has an AHI between 5 and 15, the subject has mild sleep apnea. In case the subject has an AHI between 15 and 30, the subject has moderate sleep apnea. In case the subject has an AHI of about 30, the subject has severe sleep apnea. The inventors have discovered that subjects with a high AHI and insomnia tend to have a sleep misperception representing more perceived sleep than the objective total sleep time, whereas subjects with a lower AHI and insomnia tend to have a sleep misperception representing less perceived sleep than the objective total sleep time. This insight helps to deal with the following situation: A subject has a high AHI and insomnia. Due to a change in lifestyle, the sleep condition of the subject improves over time, and as a result the subject has a lower AHI. The lower AHI is high enough for the subject to still need PAP therapy. However, due to the lower AHI and the insomnia, the subject has sleep misperception and perceives less sleep than the objective total sleep time. The PAP therapy is adjusted based on the sleep misperception. In case the PAP therapy would not be adjusted, the subject may stop with the PAP therapy in an attempt to improve the perceived amount of sleep.

In an embodiment, the computer-implemented method comprises using a further model having a further input and a further output. The further input comprises the sleep time information and the insomnia information. The further output comprises the sleep quality information. The further model is based on a further correlation between a further reference input and a further reference output. The further reference input comprises insomnia information of multiple subjects, and sleep time information during multiple sleep sessions of the multiple subjects. The further reference output comprises sleep quality information for each of the multiple sleep sessions of the multiple subjects.

The further model uses, as the further reference input, information of multiple sleep sessions of multiple subjects. For example, as reference input, information about 5 or 10 or 12 or 15 sleep sessions of one of the multiple subjects is used. Each of the multiple subjects has the same number of sleep sessions, or some of the multiple subjects have more or less sleep sessions than other ones of the multiple subjects. The further reference output comprises, for example, a sleep quality of the sleep session based on a questionnaire, such as the Consensus Sleep Diary (Carney et.al. 2012). For example, the subject may be asked to rate the sleep quality of the sleep session on a continuous scale, for example from 0 (very poor sleep quality) to 1 (excellent sleep quality). Because the further model is based on the further reference input and the further reference output, the further model is able to estimate the sleep quality information with improved accuracy.

In an embodiment, the further model comprises a beta mixed effect model.

According to this embodiment, the beta mixed effect model is used to accurately determine the sleep quality information based on the sleep time information and the insomnia information. Optionally, the beta mixed effect model is used to accurately determine the sleep quality information based on the sleep time information, the insomnia information, and the SDB information. Optionally, the beta mixed effect model is used to accurately determine the sleep quality information based on the sleep time information, the insomnia information, and at least one of the SDB information, age information of the subject, and the time the subject is awake after sleep onset. The time the subject is awake after sleep onset is known in the field as Wake After Sleep Onset, WASO.

The beta mixed effect model is mixed, because the further reference input comprises information of multiple sleep sessions for some or each of the multiple subjects.

In an embodiment, the computer-implemented method comprises generating an output signal based on the degree of sleep misperception. The output signal is representative of a suggested action to adjust an SDB therapy to treat the SDB condition of the subject.

According to this embodiment, the suggested action to adjust an SDB therapy is generated based on the degree of sleep misperception. In case the degree of sleep misperception is representative that a subject perceives to have sufficient sleep, the suggested action is, for example, to intensify the SDB therapy to reduce the number of SDB events. In case of PAP therapy, the pressure of the pressurized airflow provided to the subject is, for example, increased to intensify the therapy. In case of mandibular advancement therapy, the mandibular advancement device (MAD) is, for example, adjusted to further extend the lower jaw of the subject forward relative to the upper jaw to intensify the therapy. In case of sleep positional therapy, the level of feedback to the subject when the subject is in a target position is intensified. For example, the target position is a supine position.

In an embodiment, the SDB therapy comprises positive airway pressure (PAP) therapy. The PAP therapy comprises providing pressurized air to the subject. The suggested action comprises decreasing a pressure of the pressurized air in case the sleep misperception is representative of the subjective total sleep time being smaller than the objective total sleep time beyond a threshold.

According to this embodiment, the suggested action comprises decreasing the pressure of the pressurized air to the subject. The decrease in the pressure improves the comfort for the patient. Because of the improved comfort, the subject is less disturbed by the PAP therapy while sleeping. Because the subject is disturbed less while sleeping, the sleep quality increases. As a result, the sleep misperception is improved, enabling the subject to continue with the PAP therapy. In addition, the improved comfort may increase the objective total sleep time, because the reduction of disturbances allows for the subject to sleep longer periods without waking up.

The sleep misperception is beyond the threshold in case the subjective total sleep time is less than the subjective total sleep time defining the threshold. For example, the threshold is based on a ratio between the subjective total sleep time and the objective total sleep time. For example, the threshold is a percentage of the subjective total sleep time relative to the objective total sleep time, such as 90% or 80%. For example, the threshold is determined based on input from the subject or a caregiver. The subject or caregiver may indicate whether a certain degree of sleep misperception is acceptable or unacceptable. In case the certain degree of sleep misperception is unacceptable to the subject, the threshold is set to a degree of sleep misperception that is still acceptable. For example, the threshold comprises a plurality of thresholds. A first of the plurality of thresholds indicates, for example, that there is some degree of sleep misperception representing an underestimation of the sleep time. The degree of sleep misperception is still acceptable, so no action is suggested yet to adjust the SDB therapy. However, the subject may be monitored more intensely by the professional caretaker. A second of the plurality of thresholds indicates, for example, that there is a large degree of sleep misperception representing an underestimation of the sleep time. The degree of sleep misperception is not acceptable, so the suggested action is to adjust the SDB therapy.

In an embodiment, the suggested action comprises increasing the pressure of the pressurized air in case the sleep misperception is representative of the subjective total sleep time not being smaller than the objective total sleep time beyond the threshold.

According to the embodiment, the suggested action comprises increasing the pressure of the pressurized air to the subject. By increasing the pressure, the number of SDB events is decreased, resulting in an improved efficacy of the SDB therapy. However, the increase of the pressure may reduce the comfort for the patient. Because of the reduced comfort, the subject may be disturbed more by the PAP therapy while sleeping. Because the subject may be disturbed more while sleeping, the sleep quality may decrease. By determining the degree of sleep misperception, the SDB therapy may be balanced to provide a good efficacy to reduce the number of SDB events, while enabling the subject to sustain the SDB therapy.

In a second aspect of the invention, there is provided a computer program product, comprising instructions which, when executed by a processing system, cause the processing system to carry out the computer-implemented method of the first aspect.

In a third aspect of the invention, there is provided a respiratory support system for providing pressurized air to a subject. The respiratory support system comprises a processing system, and an interface. The processing system is configured to perform the computer-implemented method according to the first aspect. The interface is coupled to the processing system. The interface is configured to receive the sleep condition information and the sleep quality information.

In an embodiment, the processing system is configured to decrease a pressure of the pressurized air in case the sleep misperception is representative of the subjective total sleep time being smaller than the objective total sleep time beyond a threshold.

In an embodiment, the processing system is configured to increase the pressure of the pressurized air in case the sleep misperception is representative of the subjective total sleep time being not smaller than the objective total sleep time beyond the threshold.

Sleep quality is important, as a reduced sleep quality has been shown to be related to negative health effects, such as lower quality of life, and increased blood pressure. Sleep quality is a subjective value representing how a subject rates the quality of sleep of a sleep session. It is known to use a sleep diary to determine sleep quality. The sleep diary is to be filled in after each sleep session. However, a limitation of a paper sleep diary is that it is unknown when the sleep diary was filled in. A limitation of a digital sleep diary is that it may not be easy to use a digital sleep diary by subjects that have poor technological skills. Another limitation of a sleep diary is that subjects may forget to fill in the sleep diary each morning. Remembering to fill in the sleep diary may be even more difficult for elderly subjects.

Therefore, it is an objective of a fourth aspect of the invention to provide a method to improve the determination of sleep quality.

According to a second aspect of the invention, there is provided a computer-implemented method for determining sleep quality of a sleep session. The computer-implemented method comprises receiving sleep time information representative of an objective total sleep time of the subject in the sleep session. The method computer-implemented method comprises receiving insomnia information representative of insomnia of the subject. The method computer-implemented method comprises determining the sleep quality information based on the sleep time information and the insomnia information. The sleep quality is representative of a quality of sleep of the sleep session experienced by the subject.

The inventors have discovered that the sleep quality can be determined based on the sleep time information and the insomnia information. As insomnia is a sleep condition that lasts for several weeks or longer, the insomnia information needs to be determined only occasionally, such as every month or every 6 months or every year. The sleep time information is obtained by obtaining the objective total sleep time of the subject during the sleep session. The objective total sleep time is the time the subject is asleep. The objective total sleep time can be obtained via unobtrusive measurements, such as PPG or ballistocardiography. So by using the insomnia information and the objective total sleep time, the sleep quality is determined without the need for the subject to use a sleep diary. This way, an improved way of determining sleep quality is provided.

The sleep time information is representative of the objective total sleep time of the subject in the sleep session. The objective total sleep time is the sum of the time the subject is asleep during the sleep session. For example, in case the subject wakes once in the middle of the sleep session, the objective total sleep time is the sum of the sleep time prior to waking in the middle of the sleep session, and the sleep time after waking in the middle of the sleep session. No wake time during the sleep session is included in the objective total sleep time. Even if the subject is awake for only a few seconds, and has no recollection of being awake those few seconds, such short wake time is not added to the objective total sleep time.

The insomnia information is representative of insomnia of the subject. Insomnia is a sleep condition in which a subject has difficulty falling asleep and/or has difficulty staying asleep and/or wakes up early for an extended period of time. These difficulties last for at least several weeks, for example, several months or more. A subject suffering from insomnia sleeps less time than required to be fully rested after a sleep session. The presence of insomnia is, for example, determined by having the subject answer a questionnaire. Validated insomnia questionnaires are available, such as the Insomnia Severity Index. The Insomnia Severity Index has seven questions. Based on the answer to these questions, the Insomnia Severity Index outputs a severity of the insomnia as either no insomnia, subthreshold insomnia, moderate sever insomnia, and sever insomnia. The insomnia information comprises, for example, information about whether the subject has insomnia or does not have insomnia. The insomnia information comprises, for example, information about a severity of the insomnia, such as mild insomnia, moderate insomnia, and sever insomnia. For example, a subset of the questions of the Insomnia Severity Index is used, such as only the first three items. The first three items relate to a subject's difficulty falling asleep, difficulty staying asleep, and problems of waking up too early. For example, the insomnia information comprises information about insomnia symptoms experienced by the subject, such as difficulty falling asleep, difficulty staying asleep, or problems of waking up too early.

In an embodiment, the insomnia information comprises information about the insomnia based on a subjective perception of the subject.

According to this embodiment, the insomnia information comprises information based on the subjective perception of the subject. The most common way to diagnose insomnia is to ask a subject about difficulties with sleeping, and about how they feel during the day because of those difficulties. The subject responses in a subjective way to these questions, for example, by answering that falling asleep is difficult or very difficult. For example, the subject responses that there are feelings of fatigue or malaise during the day. Based on subjective perception, sleep doctors diagnose and treat insomnia. The same type of information about the subjective perception of the subject is used, in this embodiment, to determine the sleep quality. This allows for an improved accuracy of determining the sleep quality.

In an embodiment, the computer-implemented method comprises receiving sleep stage information representative of sleep stages of the subject during the sleep session. The sleep stages comprise a wake sleep stage and at least one non-wake sleep stage. The computer-implemented method comprises determining the insomnia information based on the sleep stage information.

According to this embodiment, the method makes use of sleep stage information to determine the insomnia information. The sleep stage information determines all insomnia information or only part of the insomnia information. For example, the method combines the sleep stage information with the information based on the subjective perception of the subject to determine the insomnia information. The sleep stages represent different stages of the subject during the sleep session. At least one sleep stage represents that the subject is awake, i.e., the wake sleep stage. One or more sleep stages represent that the subject is asleep. For example, there is only a single sleep stage representing that the subject is asleep. In another example, there are two sleep stages representing that the subject is asleep. One sleep stage is a Rapid Eye Movement (REM) sleep stage, whereas the other sleep stage is a non-REM sleep stage. In another example, there are three sleep stages representing that the subject is asleep. One sleep stage is a REM sleep stage, one sleep stage is a light sleep stage, and one sleep stage is a deep sleep stage. For example, the light sleep stage comprises two different sleep stages, such as N1 sleep stage and N2 sleep stage. For example, the deep sleep stage is an N3 sleep stage. For example, the sleep stage information is based on an automatic sleep stage classifier or based on manual sleep scoring.

Based on the sleep stage information, the insomnia information is determined. For example, based on the sleep stage information, the method determines how long it takes for the subject to fall asleep, how often and how long the subject is awake after sleep onset. For example, the method determines how many times the subject is awake after sleep onset for a certain period of time. The certain period of time is, for example, more than 1 minute or more than 5 minutes or more than 10 minutes. This way, the method determines the insomnia information. For example, the method determines the insomnia information by using the sleep stage information from multiple sleep sessions of the subject. For example, the multiple sleep sessions expand over a period of more than one week or more than one month.

In an embodiment, the computer-implemented method comprises receiving at least one physiological signal representative of a physiological parameter of the subject. The computer-implemented method comprises determining sleep stage information based on the at least one physiological signal.

According to this embodiment, a physiological signal is received. Based on the physiological signal, the sleep stage information is based. By using the physiological signal of the subject to determine the sleep stage information, it can be detected if the insomnia of the subject changes. For example, the insomnia becomes better or worse over time. The change of the insomnia affects the physiological parameter of the subject. The change in the physiological parameter is detected via the physiological signal.

The physiological signal is, for example, based on neurological signals of the subject. For example, the neurological signals are obtained via polysomnography (PSG), via electroencephalography (EEG), via electrooculography (EOG), via electromyography (EMG) or any combination of these. A sensor adapted to generate a sensor signal based on the neurological signals is, for example, mounted on a wearable device for the head or face, such as a headband. The sleep stage information is based on the neurological signals via use of an automated sleep stage classifier or via manual annotation.

In addition or alternatively to using neurological signals, the physiological signal is, for example, based on a surrogate measure of sleep. For example, the physiological signal comprises a cardiac signal. The cardiac signal is obtained with an appropriate sensor such as a reflective photoplethysmography (PPG) sensor, a transmissive PPG sensor or a remote PPG sensor, a ballistocardiographic sensor, or a seismocardiographic sensor. The reflective PPG sensor is, for example, arranged on the wrist or the face of the subject. The transmissive PPG sensor is, for example, arranged on the finger of the subject. The remote PPG sensor comprises, for example, an infrared camera. The ballistocardiographic sensor comprises, for example, an accelerometer or gyroscope attached to the body of the subject, or for example a pressure sensor mounted in the mattress or bed of the subject. The seismocardiographic sensor comprises, for example, an accelerometer mounted on the chest of the subject. The signals obtained by one or more of these sensors are, for example, used as input to a machine learning model trained to infer sleep stages. The input is, for example, based on manually crafted features correlating with sleep stages, such as a feature describing heart rate variability, or a feature of a time series describing heart rate progression during sleep (e.g. instantaneous heart rate). The input is, for example, input as raw data to the machine learning model.

In addition or alternatively to determining the sleep stage information as mentioned above, the sleep stage information is, for example, based on respiratory activity. Respiratory activity of the subject is indicative of changes in autonomic nervous system activity associated with different sleep stages. Respiratory activity is, for example, measured with a sensor adapted to measure airflow or adapted to measure chest movements. For example, a sensor adapted to measure airflow comprises an oral cannula, a nasal cannula and/or a thermistor. For example, a sensor adapted to measure chest movements comprises a respiratory inductance plethysmography belt to be worn around the thorax of the abdomen.

For example, the sensor adapted to measure respiratory activity comprises a pressure sensor mounted on the bed or mattress. For example, the sensor adapted to measure respiratory activity comprises a Doppler radar positioned near the subject. For example, the sensor for measuring respiratory activity comprises an accelerometer or a gyroscope mounted on the thorax, the abdomen and/or sternum of the subject.

In an embodiment, wherein the insomnia information comprises information about insomnia symptoms of the subject.

According to this embodiment, the insomnia information comprises information about insomnia symptoms, such as whether the subject has difficulty falling asleep, or has difficulty staying asleep, or has problems of waking up too early. By including insomnia symptoms in the insomnia information, an improved estimation of the sleep quality is obtained.

In an embodiment, the computer-implemented method comprises using a model having an input and an output. The input comprises the sleep time information and the insomnia information. The output comprises the sleep quality information. The model is based on a correlation between a reference input and a reference output. The reference input comprises insomnia information of multiple subjects, and sleep time information of multiple sleep sessions of the multiple subjects. The reference output comprises sleep quality information for each of the multiple sleep sessions of the multiple subjects.

The model uses, as the reference input, information of multiple sleep sessions of multiple subjects. For example, as reference input, information about 5 or 10 or 12 or 15 sleep sessions of one of the multiple subjects is used. Each of the multiple subjects has the same number of sleep sessions, or some of the multiple subjects have more or less sleep sessions than other ones of the multiple subjects. The further reference output comprises, for example, a sleep quality of the sleep session based on a questionnaire, the Consensus Sleep Diary (Carney et.al. 2012). For example, the subject may be asked to rate the sleep quality of the sleep session on a continuous scale, for example from 0 (very poor sleep quality) to 1 (excellent sleep quality). Because the model is based on the reference input and the reference output, the model is able to estimate the sleep quality information with improved accuracy.

In an embodiment, the model comprises a beta mixed effect model.

According to this embodiment, the beta mixed effect model is used to accurately determine the sleep quality information based on the sleep time information and the insomnia information. Optionally, the beta mixed effect model is used to accurately determine the sleep quality information based on the sleep time information, the insomnia information, and at least one of age information of the subject, and the time the subject is awake after sleep onset. The time the subject is awake after sleep onset is known in the field as Wake After Sleep Onset, WASO.

The beta mixed effect model is mixed, because the reference input comprises information of multiple sleep sessions for some or each of the multiple subjects.

In an embodiment, the model comprises a machine learning model. The machine learning model is at least one of a multi-level regression model, a Bayesian classifier, a logistic regression model, and a neural network.

According to this embodiment, the model comprises a machine learning model. The machine learning model is trained based on the reference input and the reference output.

In an embodiment, the computer-implemented method comprises receiving SDB information. The SDB information is representative of a sleep disordered breathing (SDB) condition of the subject based on a number of SDB events of the subject per unit of time. The computer-implemented method comprises determining the sleep quality information comprises determining the sleep quality information based on the SDB information.

The SDB information is representative of a sleep disordered breathing (SDB) condition of the subject based on a number of SDB events of the subject per unit of time. For example, the SDB information is representative of the subject having obstructive sleep apnea (OSA) or central sleep apnea (CSA). For example, the SDB events comprises events during which the subject stops breathing for longer than a certain period, such as longer than 10 seconds or 15 seconds or 20 seconds. For example, the SDB events comprises events during which the subject has reduced breathing for longer than a certain period, such as longer than 10 seconds or 15 seconds or 20 seconds. For example, the number of SDB events of the subject per unit of time is based on an apnea-hypopnea index (AHI). AHI is an index to indicate the severity of sleep apnea. AHI is calculated by dividing the number of apnea events and hypopnea events by the number of hours of sleep. For example, the AHI represents the subject has mild OSA, moderate OSA or severe OSA.

The SDB information is taken into account when determining the sleep quality information. The inventors have discovered that the SDB condition affects the sleep quality of the subject. The SDB condition is based on the number of SDB events of the subject per unit of time. In case the subject has a severe form of the SDB condition, the number of SDB events are high per unit of time. In case the subject has a mild form of the SDB condition, the number of SDB events are less high per unit of time. For example, in case the SDB condition is sleep apnea, the number of SDB events per unit of time may be represented by the Apnea-Hypopnea Index (AHI). AHI expresses the number of SDB events per hour. In case the subject has an AHI between 5 and 15, the subject has mild sleep apnea. In case the subject has an AHI between 15 and 30, the subject has moderate sleep apnea. In case the subject has an AHI of about 30, the subject has severe sleep apnea.

In an embodiment, the computer-implemented method comprises generating an output signal based on the sleep quality information. The output signal is representative of a suggested action to adjust an SDB therapy to treat a sleep disordered breathing (SDB) condition of the subject.

According to this embodiment, the suggested action to adjust the SDB therapy is generated based on the sleep quality information. In case the sleep quality is sufficiently high, the suggested action is, for example, to intensify the SDB therapy to reduce the number of SDB events. In case of PAP therapy, the pressure of the pressurized airflow provided to the subject is, for example, increased to intensify the therapy. In case of mandibular advancement therapy, the mandibular advancement device (MAD) is, for example, adjusted to further extend the lower jaw of the subject forward relative to the upper jaw to intensify the therapy. In case of sleep positional therapy, the level of feedback to the subject when the subject is in a target position is intensified. For example, the target position is a supine position. This way, the SDB therapy may be balanced to provide a good efficacy to reduce the number of SDB events, while enabling the subject to have the health benefits of a good sleep quality.

In an embodiment, the suggested action comprises improving a sleep continuity of the subject. For example, the suggested action comprises suggesting medication to improve sleep. Such medication is, for example, a hypnotic that decreases the number of awakenings and/or awake time of the subject during a sleep session. For example, the SDB therapy comprises positive airway pressure (PAP) therapy. The PAP therapy comprises providing pressurized air to the subject. The suggested action comprises decreasing a pressure of the pressurized air in case the sleep quality information represents a poor sleep quality. The decrease of the pressure improved the comfort for the patient. Because of the improved comfort, the subject is less disturbed by the PAP therapy while sleeping. Because the subject is disturbed less while sleeping, the sleep quality and the sleep continuity increases.

In an embodiment, the suggested action comprises reducing insomnia symptoms of the subject.

According to this embodiment, an action is suggested to reduce insomnia symptoms based on the sleep quality information. For example, the suggested action comprises suggesting cognitive behavioral therapy for insomnia (CBTi). For example, the suggested action comprises suggesting medication to improve sleep.

In an embodiment, the SDB therapy comprises positive airway pressure (PAP) therapy. The PAP therapy comprises providing pressurized air to the subject. The suggested action comprises decreasing a pressure of the pressurized air in case the sleep quality information is representative of a quality of sleep of the sleep session experienced by the subject below a quality threshold.

According to this embodiment, the suggested action comprises decreasing the pressure of the pressurized air to the subject. The decrease in the pressure improves the comfort for the patient. Because of the improved comfort, the subject is less disturbed by the PAP therapy while sleeping. Because the subject is disturbed less while sleeping, the objective total sleep time increases. As a result, the sleep quality increases. Because of the increased sleep quality, the subject is enabled to continue with the PAP therapy.

The sleep quality is below the threshold in case the sleep quality is unacceptable for the subject to experience. For example, the sleep quality is unacceptable to experience multiple sleep sessions in a row, for example for a week or for several weeks. For example, the threshold comprises a plurality of thresholds. A first of the plurality of thresholds indicates, for example, that the sleep quality is somewhat poor. The sleep quality is still acceptable, so no action is suggested yet to adjust the SDB therapy. However, the subject may be monitored more intensely by the professional caretaker. A second of the plurality of thresholds indicates, for example, that there is an unacceptable poor sleep quality. In case the sleep quality is less than the second threshold, the sleep quality is not acceptable, so the suggested action is to adjust the SDB therapy.

In an embodiment, the suggested action comprises increasing the pressure of the pressurized air in case the sleep quality information is representative of a quality of sleep of the sleep session experienced by the above a quality threshold.

According to this embodiment, the suggested action comprises increasing the pressure of the pressurized air to the subject. By increasing the pressure, the number of SDB events is decreased, resulting in an improved efficacy of the SDB therapy. However, the increase of the pressure may reduce the comfort for the patient. Because of the reduced comfort, the subject may be disturbed more by the PAP therapy while sleeping. Because the subject may be disturbed more while sleeping, the sleep quality may decrease. By determining the sleep quality, the SDB therapy may be balanced to provide a good efficacy to reduce the number of SDB events, while enabling the subject to sustain the SDB therapy.

In a fifth aspect of the invention, there is provided a computer program product, comprising instructions which, when executed by a processing system, cause the processing system to carry out the computer-implemented method according to the fourth aspect of the invention.

In a sixth aspect of the invention, there is provided a respiratory support system for providing pressurized air to a subject. The respiratory support the system comprises a processing system, an interface, and a sensor interface. The processing system is configured to perform the computer-implemented method according to the fourth aspect of the invention. The interface is coupled to the processing system. The sensor interface is coupled to the processing system. The interface is configured to receive the insomnia information. The sensor interface is adapted to couple to a sensor for generating a signal representative of the sleep time information. The processing system is configured to determine the sleep time information based on the sensor signal. For example, the sensor signal comprises the physiological signal representative of a physiological parameter of the subject.

BRIEF DESCRIPTION OF THE DRAWINGS

Exemplary embodiments will now be described, by way of example only, with reference to the following Figures, in which:

FIG. 1 depicts a first embodiment according to the invention;
FIG. 2 depicts a computer-implemented method according to the first embodiment;
FIG. 3 depicts a computer-implemented method according to a second embodiment,
FIG. 4 depicts a model for use in the computer-implemented method;
FIG. 5 depicts a computer-implemented method according to a third embodiment;
FIG. 6 depicts a further model for use in the computer-implemented method;
FIG. 7 depicts a computer-implemented method according to a fourth embodiment;
FIG. 8 depicts a further computer-implemented method according to a fifth embodiment;
FIG. 9 depicts a further computer-implemented method according to a sixth embodiment;
FIG. 10 depicts a further computer-implemented method according to a seventh embodiment;

DETAILED DESCRIPTION OF EMBODIMENTS

It should be understood that the detailed description and specific examples, while indicating exemplary embodiments of the systems and methods, are intended for purposes of illustration only and are not intended to limit the scope of the invention. These and other features, aspects, and advantages of the systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

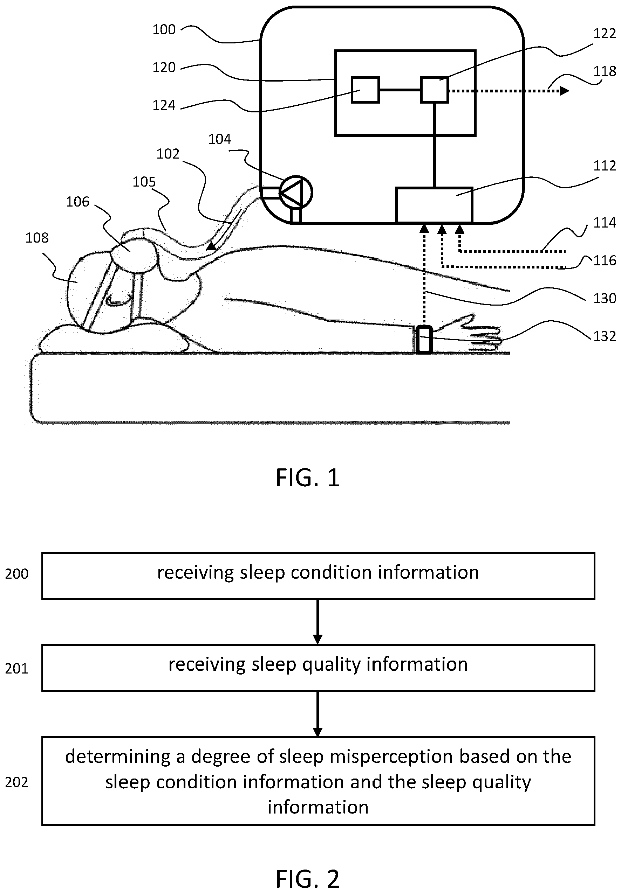

FIG. 1 depicts a first embodiment according to the invention. In the first embodiment, there is provided a respiratory support system for providing pressurized air to a subject 108. The respiratory support system 100 comprises a pressure source 104 to generate the pressurized air 102. The pressurized air 102 is directed via an air delivery tube 105 to a patient interface 106. Via the patient interface 106, the pressurized air 102 is able to prevent or help to reduce a collapse of the upper airway of the subject 108.

The respiratory support system 100 comprises a processing system 120 and an interface 112. The processing system 120 is configured to perform a computer-implemented method as explained later. The interface 112 is coupled to the processing system 120. The interface 112 is configured to receive sleep condition information 114 and sleep quality information 116.

The processing system 120 has a processor 122 and a memory 124 connected to the processor 122. The memory 124 stores a computer program product, comprising instructions which, when executed by the processor 122 of the processing system 120, cause the processing system 120 to carry out the computer-implemented method. The computer-implemented method is further explained based on FIG. 2.

The computer-implemented method is for determining sleep misperception of the subject 108 during a sleep session. The computer-implemented method comprises receiving, 200, the sleep condition information 114. The sleep condition information 114 comprises at least one of SDB information and insomnia information. The SDB information is representative of a sleep disordered breathing (SDB) condition of the subject 108 based on a number of SDB events of the subject 108 per unit of time. The insomnia information is representative of insomnia of the subject 108. The computer-implemented method comprises receiving, 201, sleep quality information 116 representative of a quality of sleep of the sleep session experienced by the subject 108. The computer-implemented method comprises determining, 202, a degree of sleep misperception based on the sleep condition information 114 and the sleep quality information 116. The sleep misperception is representative of a difference between a subjective total sleep time of the sleep session experienced by the subject 108 and an objective total sleep time of the subject 108 in the sleep session.

Optionally, the insomnia information comprises information about the insomnia based on a subjective perception of the subject 108.

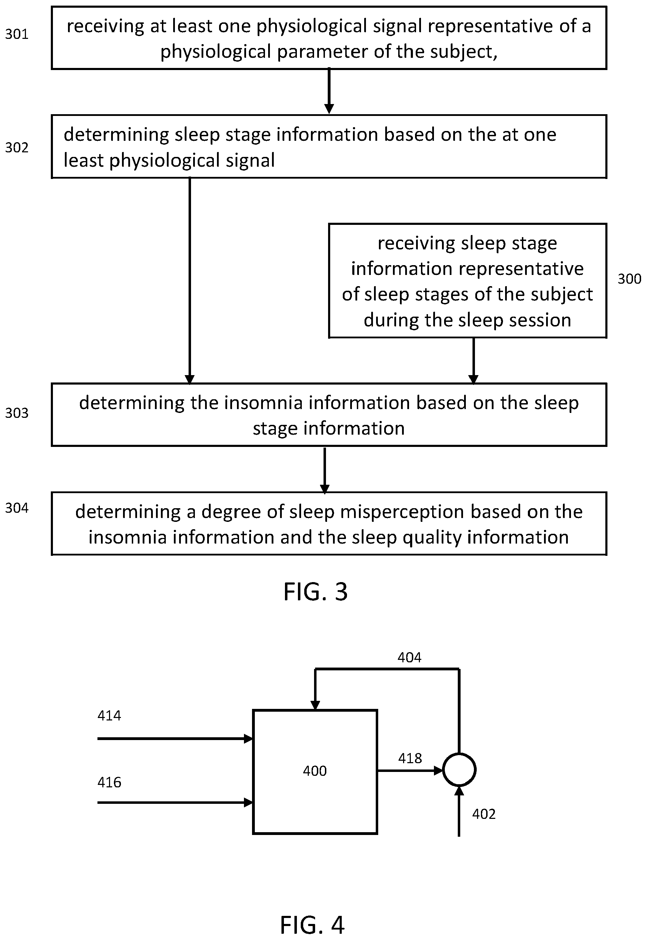

FIG. 3 depicts a computer-implemented method according to a second embodiment. The second embodiment has, for example, the same features as the first embodiment, except for the following. In the second embodiment, the computer-implemented method comprises receiving, 300, sleep stage information representative of sleep stages of the subject 108 during the sleep session. The sleep stages comprise a wake sleep stage and at least one non-wake sleep stage. The computer-implemented method comprises determining, 303, the insomnia information based on the sleep stage information. The degree of sleep misperception is determined, 304, based on the insomnia information and the sleep quality information 116.

In stead of receiving, 300, the sleep stage information, the computer-implemented method comprises, for example, receiving, 301, at least one physiological signal 130 representative of a physiological parameter of the subject 108. The physiological signal 130 is, for example, obtained via a photoplethysmography (PPG) sensor 132 arranged at the wrist of the subject 108, as depicted in FIG. 1. The computer-implemented method comprises determining sleep stage information based on the at least one physiological signal. The computer-implemented method comprises determining, 303, the insomnia information based on the sleep stage information. The degree of sleep misperception is determined, 304, based on the insomnia information and the sleep quality information 116.

FIG. 4 depicts a model 400 for use in the computer-implemented method of the first embodiment or the second embodiment. The degree of sleep misperception is determined using a model. The model 400 has an input and an output 418. The input comprises the sleep condition information 114 and the sleep quality information 116. The output 418 comprises the degree of sleep misperception. The model 400 is based on a correlation between a reference input and a reference output 402. The reference input 414 comprises sleep condition information 114 about multiple subjects. The reference input 416 comprises sleep quality information 116 about multiple sleep sessions of the multiple subjects. The reference output 402 comprises a degree of sleep misperception for each of the multiple sleep sessions of the multiple subjects. The reference output 402 is compared with the output 418 of the model 400. A difference 404 between the output 418 of the model 400 and the reference output 402 is used to determine the parameters of the model 400. In case the output 418 of the model matches with the reference output 402 within a threshold, the parameters of the model are accurately determined. As a result, the model 400 is able to accurately determine the degree of sleep misperception based on the sleep condition information 114 and the sleep quality information 116.

Optionally, the model 400 comprises a univariate linear mixed model. To generate the univariate linear mixed model, the following study was performed. A group of 79 subjects were monitored for multiple sleep sessions, with an average of 12 sleep sessions per subject. Each subject wore a wrist-worn device having a PPG sensor and an accelerometer. The objective total sleep time was derived using the information of the PPG sensor and the accelerometer in a sleep stage algorithm. The sleep stage algorithm outputs sleep stage classification for each epoch of the sleep sessions in four classes. After each sleep session, the subjective total sleep time and the sleep quality was assessed using a digital sleep diary. The SDB information was based on an ambulatory diagnostic PSG measurement. The sleep stages and the SDB events were scored by a certified sleep technician following the American Academy of Sleep Medicine (AASM) guidelines (American Academy of Sleep Medicine, 2014). A hypopnea was scored when there was an oxygen desaturation of 3% or higher and/or when the SDB event was associated with an arousal. Insomnia symptoms were defined as the presence of a nocturnal insomnia severity index (ISI) score of $\geq 4$, and at least one of sleep onset complaints and sleep maintenance complaints. The nocturnal ISI is calculated based on the first three items of the ISI. The presence of the insomnia symptoms was obtained during a clinical intake interview.

Based on the study, the linear mixed effects model was generated. The linear mixed effects model revealed the presence of insomnia symptoms, AHI, and daily reported sleep quality as significant independent predictors of the degree of sleep misperception. Specifically, the presence of insomnia symptoms is associated with a higher degree of sleep misperception in which the subjective sleep time is perceived too short, and the presence of a high AHI is associated with a higher degree of sleep misperception in which the subjective sleep time is perceived too long. Accordingly, subjects who experience insomnia symptoms tend to underestimate their total sleep time more than those subjects who do not experience insomnia symptoms. Subjects with a higher AHI tend to overestimate their total sleep time more than subjects with a lower AHI. Moreover, a lower daily reported sleep quality is associated with higher degree of sleep misperception. Thus, subjects tend to underestimate their total sleep time more after a sleep session with low sleep quality.

According to visual inspection, the distribution of residuals from the fitted linear mixed effects model was approximately normally distributed. The linear mixed effects model demonstrated an explanatory power of 0.37, as indicated by the pseudo r-squared value. This suggests that approximately 37% of the variance in the dependent variable is accounted for by the linear mixed effects model.

Table 1 represents the variables used in the linear mixed effects model for predicting the degree of sleep misperception.

| Effect type | Variables | C | SE | P |
|---|---|---|---|---|
| Constant effects | Intercept | 1.149e-01 | 6.315e-02 | 0.073 |
| | Age | -2.755e-04 | 1.120e-03 | 0.807 |
| | Sex | 8.018e-03 | 2.355e-02 | 0.735 |
| | AHI | -1.231e-03 | 5.194e-04 | 0.021* |
| | Insomnia symptoms | 8.770e-02 | 2.335e-02 | < 0.001* |

(continued)

| Effect type | Variables | C | SE | P |
|---|---|---|---|---|
| Variable effects | Reported sleep quality | -1.854e-01 | 2.944e-02 | < 0.001* |

The variables mentioned of the constant effects type, i.e., intercept, age, sex, AHI, and insomnia symptoms, were each based on one measurement per subject 108 during the clinical intake. The reported sleep quality has a variable effect, and was measured after every sleep session for every subject. "C" represents the coefficient of the predictor. "SE" represents the standard error. "p" represents the p-value. The linear mixed effects model is based on 1043 measured sleep sessions.

The significant p-values are indicated with a '*'. The variables with the significant p-values have a very strong correlation with the degree of sleep misperception. Note that the negative sign of "C" for the AHI-variable indicates that the degree of sleep misperception becomes less with an increase of AHI.

Optionally, the model 400 comprises a machine learning model. For example, the machine learning model is at least one of a multi-level regression model, a Bayesian classifier, a logistic regression model, and a neural network.

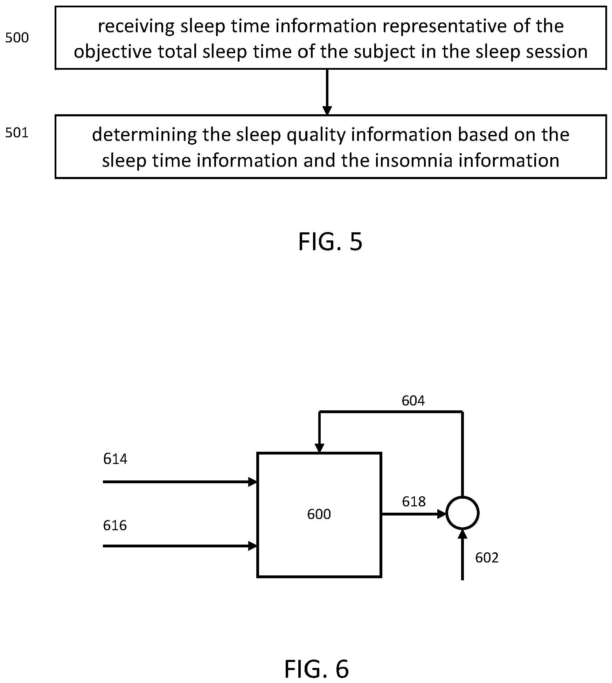

FIG. 5 depicts a computer-implemented method according to a third embodiment. The third embodiment has, for example, the same features as the first embodiment or as the second embodiment, except for the following. In the third embodiment, the computer-implemented method comprises receiving, 500, sleep time information representative of the objective total sleep time of the subject 108 in the sleep session, and determining, 501, the sleep quality information 116 based on the sleep time information and the insomnia information. Optionally, determining the sleep quality information 116 comprises determining the sleep quality information 116 based on the SDB information.

FIG. 6 depicts a further model 600 for use in the computer-implemented method of the first embodiment or the second embodiment or the third embodiment. The further model 600 has a further input and a further output 618. The further input comprises the sleep time information and the insomnia information. The further output 618 comprises the sleep quality information 116. The further model 600 is based on a further correlation between a further reference input and a further reference output 602. The further reference input 614 comprises insomnia information of multiple subjects. The further reference input 616 comprises sleep time information during multiple sleep sessions of the multiple subjects. The further reference output 602 comprises sleep quality information 116 for each of the multiple sleep sessions of the multiple subjects.

The reference output 602 is compared with the output 618 of the further model. A difference 604 between the output 618 of the further model 600 and the reference output 602 is used to determine the parameters of the further model. In case the output 618 of the further model 600 matches with the reference output 602 within a threshold, the parameters of the further model 600 are accurately determined. As a result, the further model 600 is able to accurately determine the sleep quality information 116 based on the insomnia information and the sleep time information.

Optionally, the further model 600 comprises a beta mixed effect model. To generate the beta mixed effect model, the following study was performed.

In the study, 135 subjects having OSA, which is a sleep condition, were monitored for multiple sleep sessions, with an average of 12 sleep sessions for each subject. Each subject used a sleep diary. Sleep quality was measured with a daily question in the sleep diary, developed by Sleep Medicine Center Kempenhaeghe, Heeze, the Netherlands. The sleep diary was an application on a smartphone given to each subject. The subjects were asked to fill in the sleep diary in the morning. Sleep quality was measured with the question: 'What was the quality of your sleep?'. Subjects subjectively indicated the sleep quality on a continuous line ranking from 'very bad' to 'excellent' with a slider. Sleep quality was quantified in a continuous scale from zero ('very bad') to one ('excellent' sleep quality). Objective sleep variables were estimated from wrist-worn measurements of PPG and accelerometery. The resting period was detected based on accelerometer signal. In the detected resting period, sleep stages were automatically classified into four stages (Wake, N1/N2, N3, REM) based on instantaneous heart rate derived from PPG, and on gross estimates of body movements derived from accelerometer, using an algorithm developed and validated in sleep disordered populations. Based on the detected sleep stages, sleep statistics such as total sleep time (TST), wake after sleep onset (WASO), sleep onset latency (SOL) and number of awakenings (WKN) were determined. The total sleep time was calculated as the total resting period minus wake time during the resting period. WASO was calculated as total wake time between first and final epoch of sleep of that resting period. SOL was determined as the time from the beginning of the resting period to the first epoch of the first series of three consecutive epochs of N1/N2, or any shorter sequence of non-Wake epochs of containing N3 or REM. WKN was determined as total number of awakenings during the night, where awakening was defined as the first of a series of wake epochs of any duration. Insomnia symptoms were assessed by the first three items of the insomnia severity index (ISI) questionnaire. AHI was used from a diagnostic session with a polysomnography (PSG). During the diagnostic session, an apnea was scored when there was a decrease in airflow by 90% or more of pre-event baseline, for 10 seconds or longer. A hypopnea was scored when there was a decrease between 30% and 90% of pre-event baseline, and the event was associated with an oxygen desaturation of 3% or higher and/or when the SDB event was associated with an arousal. Age and sex were retrieved from the medical records of the subjects.

The sleep qualities after every sleep session were transformed to obtain open-ended intervals, according to the following formula: y_transformed = (y(N-1)+0.5)/N (Smithson & Verkuilen, 2006), with y corresponding to a value of sleep quality after a sleep session, y_transformed the transformed datapoint and N the number of samples. A beta mixed effect model was created with the sleep quality after a sleep session set as outcome variable. Age, sex, AHI, insomnia symptoms, TST, WASO, SOL, WKN and the day of the week "weekday" were included in the model.

Results showed that a higher sleep quality for a sleep session was associated with longer total sleep time during the sleep session. (estimate = 0.119 (0.022), p<0.001) and with shorter WASO during the sleep session (estimate = -0.177 (0.0221), p<0.001). SOL and WKN of a sleep session did not seem to be significantly related to daily subjective sleep quality. In addition, a lower nocturnal ISI score was related to higher sleep quality (estimate = -0.335 (0.057), p<0.001). Next to that, higher sleep quality was associated with higher AHI (estimate = 0.131 (0.057), p = 0.021) and higher age (estimate = 0.149 (0.057), p = 0.009). The pseudo-R squared of the model was 0.5853, which means that the model explained 58.53% of the variance of sleep quality after a sleep session.

Table 2 represents the variables used in the beta mixed effects model for predicting the sleep quality.

|  | Variables | C | SE | p-value |
|---|---|---|---|---|
|  | Intercept | 0.223 | 0.100 | 0.026* |
| Constant effects | Age (years) | 0.149 | 0.057 | 0.009* |
|  | Sex (male/female) | 0.083 | 0.121 | 0.494 |
|  | AHI (events/hour) | 0.131 | 0.057 | 0.021* |
|  | Nocturnal ISI score | -0.335 | 0.057 | <0.001* |
| Variable effects | Weekday | 0.015 | 0.037 | 0.695 |
|  | WASO (min) | -0.177 | 0.022 | <0.001* |
|  | SOL (min) | -0.011 | 0.019 | 0.577 |
|  | TST (min) | 0.119 | 0.022 | <0.001* |
|  | WKN | 0.023 | 0.026 | 0.386 |

The variables with a variable effect were measured with every sleep session. The variables with the variable effect are 'weekday', WASO, SOL, total sleep time TST, and WKN. "C" represents the coefficient of the predictor. "SE" represents the standard error. "p" represents the p-value.

The significant p-values are indicated with a '*'. The corresponding variables have a very strong correlation with the sleep quality.

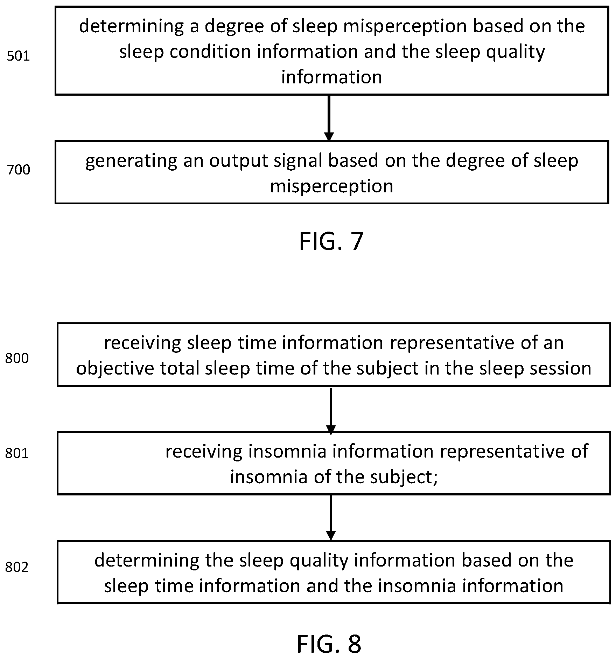

FIG. 7 depicts a computer-implemented method according to a fourth embodiment. The fourth embodiment has, for example, the same features as the first embodiment, the second embodiment, or the third embodiment, except for the following.

In the fourth embodiment, the computer-implemented method comprises generating, 700, an output signal based on the degree of sleep misperception. The output signal is representative of a suggested action to adjust an SDB therapy to treat the SDB condition of the subject 108.

For example, the SDB therapy comprises positive airway pressure (PAP) therapy, such as provided by the respiratory support system 100. The PAP therapy comprises providing pressurized air 102 to the subject 108. The suggested action comprises decreasing a pressure of the pressurized air 102 in case the sleep misperception is representative of the subjective total sleep time being smaller than the objective total sleep time beyond a threshold.

Optionally, the suggested action comprises increasing the pressure of the pressurized air 102 in case the sleep misperception is representative of the subjective total sleep time not being smaller than the objective total sleep time beyond the threshold.

Optionally, the processing system 120 is configured to decrease a pressure of the pressurized air 102 in case the sleep misperception is representative of the subjective total sleep time being smaller than the objective total sleep time beyond a threshold.

Optionally, the processing system 120 is configured to increase the pressure of the pressurized air 102 in case the sleep misperception is representative of the subjective total sleep time being not smaller than the objective total sleep time beyond the threshold.

In a fifth embodiment, the respiratory support system 100 according to the first embodiment is provided. In the fifth embodiment, the memory 124 stores a computer program product, comprising instructions which, when executed by the processor 122 of the processing system 120, cause the processing system 120 to carry out a further computer-implemented method. The further computer-implemented method is explained based on FIG. 8.

FIG. 8 depicts a further computer-implemented method according to the fifth embodiment. The further computer-implemented method is for determining sleep quality of a sleep session. The further computer-implemented method comprises receiving, 800, sleep time information representative of an objective total sleep time of the subject 108 in the sleep session. The further computer-implemented method comprises receiving, 801, insomnia information representative of insomnia of the subject 108. The further computer-implemented method comprises determining, 802, the sleep quality information 116 based on the sleep time information and the insomnia information. The sleep quality is representative of a quality of sleep of the sleep session experienced by the subject 108.

Optionally, the insomnia information comprises information about the insomnia based on a subjective perception of the subject 108.

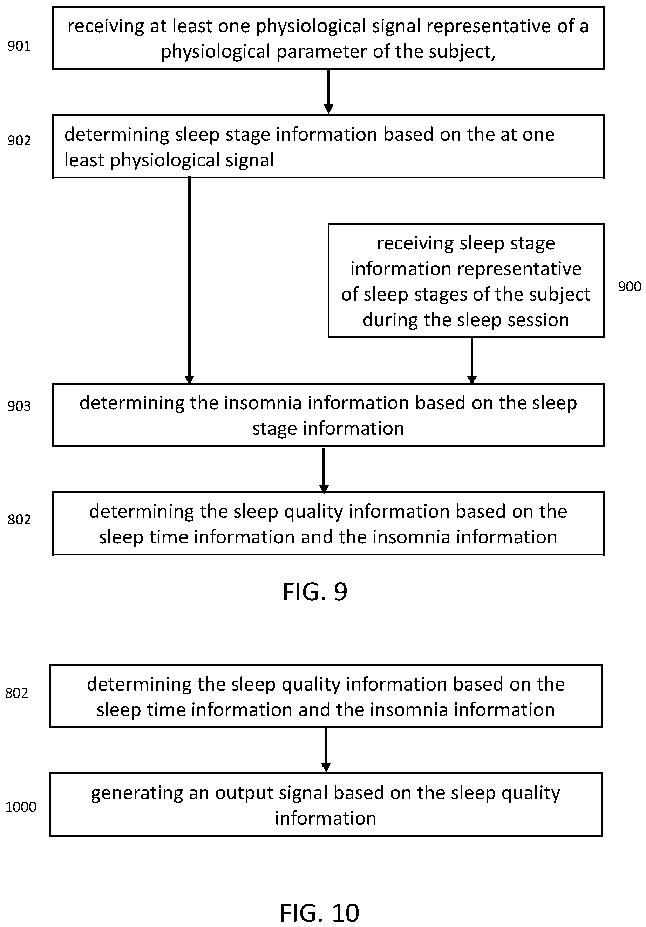

FIG. 9 depicts the further computer-implemented method according to a sixth embodiment. The sixth embodiment has, for example, the same features as the fifth embodiment, except for the following. In the sixth embodiment, the computer-implemented method comprises receiving, 900, sleep stage information representative of sleep stages of the subject 108 during the sleep session. The sleep stages comprise a wake sleep stage and at least one non-wake sleep stage. The computer-implemented method comprises determining, 903, the insomnia information based on the sleep stage information. The computer-implemented method comprises determining, 802, the sleep quality information based on the sleep time information and the insomnia information.

In stead of receiving, 900, the sleep stage information, the further computer-implemented method comprises, for example, receiving, 901, at least one physiological signal 130 representative of a physiological parameter of the subject 108. The physiological signal 130 is, for example, obtained via a photoplethysmography (PPG) sensor 132 arranged at the wrist of the subject 108. The computer-implemented method comprises determining, 902, sleep stage information based on the at least one physiological signal 130. The further computer-implemented method comprises determining, 903, the insomnia information based on the sleep stage information. The further computer-implemented method comprises determining, 802, the sleep quality information based on the sleep time information and the insomnia information.

Optionally, the insomnia information comprises information about insomnia symptoms of the subject 108.

In a seventh embodiment, the further computer-implemented method makes use of the further model 600 as depicted in FIG. 6. The further model 600 has a further input and a further output 618. The further input comprises the sleep time information and the insomnia information. The further output 618 comprises the sleep quality information 116. The further model 600 is based on a further correlation between a further reference input and a further reference output 602. The further reference input 614 comprises insomnia information of multiple subjects. The further reference input 616 comprises sleep time information during multiple sleep sessions of the multiple subjects. The further reference output 602 comprises sleep quality information 116 for each of the multiple sleep sessions of the multiple subjects.

The reference output 602 is compared with the output 618 of the further model 600. A difference 604 between the output 618 of the further model 600 and the reference output 602 is used to determine the parameters of the further model 600. In case the output 618 of the further model matches with the reference output 602 within a threshold, the parameters of the further model 600 are accurately determined. As a result, the further model 600 is able to accurately determine the sleep quality information 116 based on the insomnia information and the sleep time information.

Optionally, the further model 600 comprises a beta mixed effect model. The beta mixed model is, for example, generated based on the study and the parameters relating to Table 2.

Optionally, the further model 600 comprises a machine learning model. For example, the machine learning model is at least one of a multi-level regression model, a Bayesian classifier, a logistic regression model, and a neural network.

Optionally, the further computer-implemented method comprises receiving SDB information. The SDB information is representative of a sleep disordered breathing (SDB) condition of the subject 108 based on a number of SDB events of the subject 108 per unit of time. Determining the sleep quality information 116 comprises determining the sleep quality information 116 based on the SDB information.

FIG. 10 depicts a further computer-implemented method according to a seventh embodiment. The seventh embodiment has, for example, the same features as the first embodiment, the fifth embodiment, or the sixth embodiment, except for the following. The further computer-implemented method comprises generating, 1000, an output signal based on the sleep quality information 116. The output signal is representative of a suggested action to adjust an SDB therapy to treat a sleep disordered breathing (SDB) condition of the subject 108.

For example, the suggested action comprises improving a sleep continuity of the subject 108.

For example, the suggested action comprises reducing insomnia symptoms of the subject 108.

For example, the SDB therapy comprises positive airway pressure (PAP) therapy, such as provided by the respiratory support system 100. The PAP therapy comprises providing pressurized air 102 to the subject 108. The suggested action comprises decreasing a pressure of the pressurized air 102 in case the sleep quality information 116 is representative of a quality of sleep of the sleep session experienced by the subject 108 below a quality threshold.

The suggested action comprises increasing the pressure of the pressurized air 102 in case the sleep quality information 116 is representative of a quality of sleep of the sleep session experienced by the above a quality threshold.

In a further embodiment, the respiratory support system 100 is for providing pressurized air 102 to a subject 108. The respiratory support system 100 comprises the processing system 120, the interface 112, and a sensor interface. The processing system 120 is configured to perform the further computer-implemented method according to any one of the fifth embodiment, the sixth embodiment or the seventh embodiment. The interface 112 is coupled to the processing system 120. The sensor interface is coupled to the processing system 120. The interface 112 is configured to receive the insomnia information. The sensor interface is adapted to couple to a sensor for generating a signal representative of the sleep time information. The processing system 120 is configured to determine the sleep time information based on the sensor signal. For example, the signal from the sensor is the physiological signal 130 representative of a physiological parameter of the subject 108.

Embodiments according to the invention are presented in the following clauses.

Clause 1: A computer-implemented method for determining sleep quality of a sleep session, the computer-implemented method comprising:

receiving sleep time information representative of an objective total sleep time of the subject 108 in the sleep session;
receiving insomnia information representative of insomnia of the subject 108;
determining the sleep quality information 116 based on the sleep time information and the insomnia information, wherein the sleep quality is representative of a quality of sleep of the sleep session experienced by the subject 108.

Clause 2: The computer-implemented method according to clause 1, wherein the insomnia information comprises information about the insomnia based on a subjective perception of the subject 108.

Clause 3: The computer-implemented method according to any one of clauses 1 or 2, comprising:

receiving sleep stage information representative of sleep stages of the subject 108 during the sleep session, wherein the sleep stages comprise a wake sleep stage and at least one non-wake sleep stage;
determining the insomnia information based on the sleep stage information.

Clause 4: The computer-implemented method according to clause 3, comprising receiving at least one physiological signal representative of a physiological parameter of the subject 108, determining sleep stage information based on the at least one physiological signal.

Clause 5: The computer-implemented method according to any one of clause 1-4, wherein the insomnia information comprises information about insomnia symptoms of the subject 108.

Clause 6: The computer-implemented method according to any one of clauses 1-5, comprising: using a model having an input and an output,

wherein the input comprises the sleep time information and the insomnia information,
wherein the output comprises the sleep quality information 116,
wherein the model is based on a correlation between a reference input and a reference output,
wherein the reference input comprises insomnia information of multiple subjects, and sleep time information of multiple sleep sessions of the multiple subjects,
wherein the reference output comprises sleep quality information 116 for each of the multiple sleep sessions of the multiple subjects.

Clause 7: The computer-implemented method according to clause 6, wherein the model comprises a beta mixed effect model.

Clause 8: The computer-implemented method according to clause 6 or 7, wherein the model comprises a machine learning model, wherein the machine learning model is at least one of a multi-level regression model, a Bayesian classifier, a logistic regression model, and a neural network.

Clause 9: The computer-implemented method according to any one of clauses 1-8, comprising receiving SDB information,

wherein the SDB information is representative of a sleep disordered breathing (SDB) condition of the subject 108 based on a number of SDB events of the subject 108 per unit of time,
wherein determining the sleep quality information 116 comprises determining the sleep quality information 116 based on the SDB information.

Clause 10: The computer-implemented method according to any one of clauses 1-9, comprising generating an output signal based on the sleep quality information 116,
wherein the output signal is representative of a suggested action to adjust an SDB therapy to treat a sleep disordered breathing (SDB) condition of the subject 108.

Clause 11: The computer-implemented method according to clause 10, wherein the suggested action comprises improving a sleep continuity of the subject 108.

Clause 12: The computer-implemented method according to clause 10 or 11, wherein the suggested action comprises reducing insomnia symptoms of the subject 108.

Clause 13: The computer-implemented method according to any one of clauses 10-12, wherein the SDB therapy comprises positive airway pressure (PAP) therapy,

wherein the PAP therapy comprises providing pressurized air to the subject 108,
wherein the suggested action comprises decreasing a pressure of the pressurized air in case the sleep quality information 116 is representative of a quality of sleep of the sleep session experienced by the subject 108 below a quality threshold.

Clause 14: The computer-implemented method according to any one of clauses 10-13, wherein the suggested action comprises increasing the pressure of the pressurized air in case the sleep quality information 116 is representative of a quality of sleep of the sleep session experienced by the above a quality threshold.

Clause 15: A computer program product, comprising instructions which, when executed by a processing system 120, cause the processing system 120 to carry out the computer-implemented method of clauses 1-14.

Clause 16: A respiratory support system for providing pressurized air to a subject 108, the respiratory support system 100 comprising:

a processing system 120 configured to perform the computer-implemented method according to any one of clauses 1-14;
an interface 112 coupled to the processing system 120;
a sensor interface 112 coupled to the processing system 120,
wherein the interface 112 is configured to receive the insomnia information,
wherein the sensor interface 112 is adapted to couple to a sensor for generating a signal representative of the sleep time information,
wherein the processing system 120 is configured to determine the sleep time information based on the sensor signal.

It will be understood that the disclosed methods are computer-implemented methods. As such, there is also proposed a concept of a computer program comprising code means for implementing any described method when said program is run on a processing system 120.

The skilled person would be readily capable of developing a processing system 120 for carrying out any herein described method. Thus, each step of a flow chart may represent a different action performed by a processing system 120, and may be performed by a respective module of the processing system 120.

As discussed above, the system makes use of a processing system 120 to perform the data processing. The processing system 120 can be implemented in numerous ways, with software and/or hardware, to perform the various functions required. The processing system 120 typically employs one or more microprocessors that may be programmed using software (e.g. microcode) to perform the required functions. The processing system 120 may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs). Thus, the processing system 120 may be embodied as a digital and/or analog processing system 120.

In various implementations, the processing system 120 may be associated with one or more storage media such as volatile and non-volatile computer memory 124 such as RAM, PROM, EPROM, and EEPROM. The storage media may be encoded with one or more programs that, when executed on one or more processing systems and/or controllers, perform the required functions. Various storage media may be fixed within a processing system 120 or controller may be transportable, such that the one or more programs stored thereon can be loaded into a processing system 120.

Functions implemented by a processor 122 may be implemented by a single processor 122 or by multiple separate processing units which may together be considered to constitute a "processor". Such processing units may in some cases be remote from each other and communicate with each other in a wired or wireless manner.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

1.  A computer-implemented method for determining sleep misperception of a subject (108) during a sleep session, the computer-implemented method comprising:

    receiving sleep condition information (114),
    wherein the sleep condition information (114) comprises at least one of SDB information and insomnia information,
    wherein the SDB information is representative of a sleep disordered breathing (SDB) condition of the subject (108) based on a number of SDB events of the subject (108) per unit of time,
    wherein the insomnia information is representative of insomnia of the subject (108);
    receiving sleep quality information (116) representative of a quality of sleep of the sleep session experienced by the subject (108);
    determining a degree of sleep misperception based on the sleep condition information (114) and the sleep quality information (116),
    wherein the sleep misperception is representative of a difference between a subjective total sleep time of the sleep session experienced by the subject (108) and an objective total sleep time of the subject (108) in the sleep session.

2.  The computer-implemented method according to claim 1, wherein the insomnia information comprises information about the insomnia based on a subjective perception of the subject (108).

3.  The computer-implemented method according to claim 1 or 2, comprising:

    receiving sleep stage information representative of sleep stages of the subject (108) during the sleep session,
    wherein the sleep stages comprise a wake sleep stage and at least one non-wake sleep stage;
    determining the insomnia information based on the sleep stage information.

4.  The computer-implemented method according to claim 3, comprising receiving at least one physiological signal (130) representative of a physiological parameter of the subject (108); and
    determining sleep stage information based on the at least one physiological signal (130).

5.  The computer-implemented method according any one of the preceding claims,

    wherein determining the degree of sleep misperception comprises:
    using a model (400) having an input and an output,
    wherein the input comprises the sleep condition information (114) and the sleep quality information (116),
    wherein the output (418) comprises the degree of sleep misperception,
    wherein the model (400) is based on a correlation between a reference input and a reference output,
    wherein the reference input comprises sleep condition information (414) about multiple subjects,
    wherein the reference input comprises sleep quality information (416) about multiple sleep sessions of the multiple subjects,
    wherein the reference output (402) comprises a degree of sleep misperception for each of the multiple sleep sessions of the multiple subjects.

6.  The computer-implemented method according to claim 5, wherein the model (400) comprises a univariate linear mixed model.

7.  The computer-implemented method according to any one of the preceding claims, comprising:

receiving sleep time information representative of the objective total sleep time of the subject (108) in the sleep session;

determining the sleep quality information (116) based on the sleep time information and the insomnia information.

8. The computer-implemented method according to claim 7, wherein determining the sleep quality information (116) comprises determining the sleep quality information (116) based on the SDB information.

9. The computer-implemented method according to claim 7 or 8, comprising using a further model (600) having a further input and a further output (618),

wherein the further input comprises the sleep time information and the insomnia information,
wherein the further output (618) comprises the sleep quality information (116),
wherein the further model is based on a further correlation between a further reference input (614, 616) and a further reference output (602),
wherein the further reference input (614) comprises insomnia information of multiple subjects,
wherein the further reference input (616) comprises sleep time information during multiple sleep sessions of the multiple subjects, and
wherein the further reference output (602) comprises sleep quality information (116) for each of the multiple sleep sessions of the multiple subjects.

10. The computer-implemented method according to claim 9, wherein the further model (600) comprises a beta mixed effect model.

11. The computer-implemented method according to any one of the preceding claims, comprising:

generating an output signal based on the degree of sleep misperception,
wherein the output signal is representative of a suggested action to adjust an SDB therapy to treat the SDB condition of the subject (108).

12. The computer-implemented method according to claim 11, wherein the SDB therapy comprises positive airway pressure (PAP) therapy,

wherein the PAP therapy comprises providing pressurized air (102) to the subject (108),
wherein the suggested action comprises decreasing a pressure of the pressurized air (102) in case the sleep misperception is representative of the subjective total sleep time being smaller than the objective total sleep time beyond a threshold.

13. A computer program product, comprising instructions which, when executed by a processing system (120), cause the processing system (120) to carry out the computer-implemented of claims 1-12.

14. A respiratory support system (100) for providing pressurized air (120) to a subject (108), the respiratory support system (100) comprising:

a processing system (120) configured to perform the computer-implemented method according to any one of claims 1-12; and
an interface (112) coupled to the processing system (120),
wherein the interface (112) is configured to receive the sleep condition information (114) and the sleep quality information (116).

15. The respiratory support system (100) according to claim 14, wherein the processing system (120) is configured to decrease a pressure of the pressurized air (120) in case the sleep misperception is representative of the subjective total sleep time being smaller than the objective total sleep time beyond a threshold.

FIG. 1

200 — receiving sleep condition information

201 — receiving sleep quality information

202 — determining a degree of sleep misperception based on the sleep condition information and the sleep quality information

FIG. 2

| 301 | receiving at least one physiological signal representative of a physiological parameter of the subject, |
|---|---|

| 302 | determining sleep stage information based on the at one least physiological signal |
|---|---|

| | receiving sleep stage information representative of sleep stages of the subject during the sleep session | 300 |
|---|---|---|

| 303 | determining the insomnia information based on the sleep stage information |
|---|---|

| 304 | determining a degree of sleep misperception based on the insomnia information and the sleep quality information |
|---|---|

## FIG. 3

404

414

416

400

418

402

## FIG. 4

| 500 | receiving sleep time information representative of the objective total sleep time of the subject in the sleep session |
|---|---|

| 501 | determining the sleep quality information based on the sleep time information and the insomnia information |
|---|---|

FIG. 5

614

616

600

604

618

602

FIG. 6

determining a degree of sleep misperception based on the sleep condition information and the sleep quality information

generating an output signal based on the degree of sleep misperception

## FIG. 7

receiving sleep time information representative of an objective total sleep time of the subject in the sleep session

receiving insomnia information representative of insomnia of the subject;

determining the sleep quality information based on the sleep time information and the insomnia information

## FIG. 8

901 — receiving at least one physiological signal representative of a physiological parameter of the subject,

902 — determining sleep stage information based on the at one least physiological signal

receiving sleep stage information representative of sleep stages of the subject during the sleep session

900

903 — determining the insomnia information based on the sleep stage information

802 — determining the sleep quality information based on the sleep time information and the insomnia information

FIG. 9

802 — determining the sleep quality information based on the sleep time information and the insomnia information

1000 — generating an output signal based on the sleep quality information

FIG. 10

Europäisches
Patentamt

European
Patent Office

Office européen
des brevets

# EUROPEAN SEARCH REPORT

**Application Number**

EP 24 16 9585

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/347412 A1 (SHOULDICE REDMOND [IE]) 3 November 2022 (2022-11-03) | 1-4,7,8, 13,14 | INV. G16H20/30 |
| A | * paragraphs [0048], [0049], [0081], [0100], [0109]; figure 1 * ----- | 5,6, 9-12,15 | A61B5/00 G16H50/20 |
| A | WO 2023/031737 A1 (RESMED SENSOR TECH LTD [IE]) 9 March 2023 (2023-03-09) * paragraphs [0020] - [0079] * ----- | 1-15 | |
| A | CHOI SU JUNG ET AL: "Discordant sleep parameters among actigraphy, polysomnography, and perceived sleep in patients with sleep-disordered breathing in comparison with patients with chronic insomnia disorder", SLEEP AND BREATHING - SCHLAF UND ATMUNG, DRUCKBILD, TITISEE-NEUSTADT, DE, vol. 21, no. 4, 14 June 2017 (2017-06-14), pages 837-843, XP036371206, ISSN: 1520-9512, DOI: 10.1007/S11325-017-1514-5 [retrieved on 2017-06-14] * the whole document * ----- | 1-15 | |

**TECHNICAL FIELDS SEARCHED (IPC)**

G16H
A61B

The present search report has been drawn up for all claims

| Place of search | Date of completion of the search | Examiner |
|---|---|---|
| Munich | 26 August 2024 | Rivera Pons, Carlos |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

                                                                     

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 16 9585

This annex lists the patent family members relating to the patent documents cited in the above-mentioned European search report.
The members are as contained in the European Patent Office EDP file on
The European Patent Office is in no way liable for these particulars which are merely given for the purpose of information.

26-08-2024

| Patent document cited in search report | Publication date | Patent family member(s) | | Publication date |
|---|---|---|---|---|
| US 2022347412 A1 | 03-11-2022 | AU | 2020373407 A1 | 26-05-2022 |
| | | AU | 2023201804 A1 | 27-04-2023 |
| | | CN | 114901134 A | 12-08-2022 |
| | | CN | 117084631 A | 21-11-2023 |
| | | EP | 4051091 A1 | 07-09-2022 |
| | | EP | 4252631 A2 | 04-10-2023 |
| | | JP | 2023500515 A | 06-01-2023 |
| | | US | 2022347412 A1 | 03-11-2022 |
| | | US | 2023191057 A1 | 22-06-2023 |
| | | WO | 2021084478 A1 | 06-05-2021 |
| WO 2023031737 A1 | 09-03-2023 | CN | 118202422 A | 14-06-2024 |
| | | EP | 4396832 A1 | 10-07-2024 |
| | | WO | 2023031737 A1 | 09-03-2023 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CARNEY**. *Consensus Sleep Diary*, 2012

- American Academy of Sleep Medicine (AASM) guidelines. American Academy of Sleep Medicine, 2014